# EUROPEAN PATENT APPLICATION

(11) **EP 3 795 166 A1**
(43) Date of publication of application: **24.03.2021**
(21) Application number: 19803071.0
(22) Date of filing: 15.05.2019
(51) Int. Cl.: A61K 38/07, A61P 29/00

(54) **PHARMACEUTICAL COMPOSITION OF KOR RECEPTOR AGONIST**

(30) Priority: 16.05.2018 CN 201810469196
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN); Suncadia Pharmaceuticals Co., Ltd, Chengdu, Sichuan 610000 (CN)
(72) Inventor: TONG, Xinyong, Shanghai 200245 (CN); ZOU, Aifeng, Shanghai 200245 (CN); ZHOU, Yin, Shanghai 200245 (CN); FAN, Yi, Shanghai 200245 (CN); TAO, Weikang, Shanghai 200245 (CN)
(74) Representative: Viganò, Elena
(86) International application number: PCT/CN2019/086984
(87) International publication number: WO 2019/219019

(57) **Abstract**

Disclosed in the present invention is a pharmaceutical composition of a KOR receptor agonist, which comprises 4-amino-N-[N2-[N-[N-[N-((R)-2-phenyl propyl)glycyl]-D-phenylalanyl]-D-leucyl]-D-lysyl]piperidine-4-carboxylic acid or available salts thereof, and an acetate buffer solution.

## Description

### FIELD OF THE INVENTION

The present disclosure belongs to the field of pharmaceutical formulations, and relates to a pharmaceutical composition of a KOR receptor agonist 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid and a method for preparing the same.

### BACKGROUND OF THE INVENTION

κ-Opioid receptor (KOR) exists in the brain, spinal cord, central and peripheral nerve endings, (somatic and visceral) primary sensory afferent nerve cells, and immune cells. It involves in important physiological activities such as pain perception, neuroendocrine, emotional behavior and cognition.

Jolivalt et al. (Diabetologia 2006,49(11):2775-85) report the effect of the KOR agonist asimadoline in rodent diabetic neuropathy. Bileviciute-Ljungar et al. (Eur. J. Pharm 2004.494:139-46) report the effect of the KOR agonist U-50488 in chronic compressive injury (CCI) model in rats with neuropathic pain and the blockade of the opioid antagonist naloxone on its effect. Riviere, Br reports the use of KOR agonists in the treatment or prevention of visceral pain, including gynecological conditions such as dysmenorrhea and endometriosis (J. Pharmacol 2004.141:1331-4). These observations support the use of KOR agonists in the treatment of neuropathic pain caused by diabetes, viruses and chemotherapy.

The currently disclosed patent applications related to KOR receptor agonist include WO2007139826, WO2008060552, WO09932510, WO2013184794, WO2014089019, WO2014184356 and WO2015065867. In addition, WO2017211272 also discloses a novel KOR receptor agonist, *i.e.,* 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid.

Since 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid has a low solubility, there is a need to develop a pharmaceutical composition (formulation) of the KOR receptor agonist that can facilitate its administration, keep stability during storage and subsequent use, and show better effects.

### SUMMARY OF THE INVENTION

The present disclosure provides a pharmaceutical composition comprising the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof and a buffer solution.

In alternative embodiments, the buffer solution of the present disclosure is an acetate buffer solution, and preferably an acetic acid-sodium acetate buffer solution.

In alternative embodiments, the pH of the pharmaceutical composition is about 2.0 to 6.0, can be about 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9 or 6.0, and preferably about 3.0 to 5.0.

In alternative embodiments, the concentration of the buffer solution in the pharmaceutical composition is about 1 to 150 mM, can be about 1, 5, 10, 15, 20, 25, 30, 35, 40, 45, 50, 55, 60, 65, 70, 75, 80, 85, 90, 95, 100, 105, 110, 115, 120, 125, 130, 135, 140, 145 or 150 mM, preferably about 10 to 80 mM, and most preferably about 20 mM.

The concentration of the active ingredient or a pharmaceutically acceptable salt thereof in the pharmaceutical composition of the present disclosure is about 0.1 to 1.0 mg/ml, and can be about 0.1, 0.2, 0.3, 0.4, 0.5, 0.6, 0.7, 0.8, 0.9 or 1.0 mg/ml.

Further, the osmotic pressure of the pharmaceutical composition of the present disclosure is about 280 to 320 mOsmol/kg, and non-limiting examples include 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319 or 320 mOsmol/kg.

In alternative embodiments, the pharmaceutical composition also comprises a saccharide. The term "saccharide" of the present disclosure comprises conventional compositions (CH2O)ₙ and derivatives thereof, including a monosaccharide, disaccharide, trisaccharide, polysaccharide, sugar alcohol, reducing sugar, non-reducing sugar and the like. The saccharide can be selected from glucose, sucrose, trehalose, lactose, fructose, maltose, dextran, glycerin, erythritol, glycerol, arabitol, sylitol, sorbit, mannitol, melibiose, melezitose, raffinose, mannotriose, stachyose, maltose, lactulose, maltulose, sorbitol, maltitol, lactitol, *iso*-maltulose and the like, and preferably mannitol.

In alternative embodiments, the concentration of the saccharide in the pharmaceutical composition is about 2 to 100 mg/ml, and non-limiting examples include 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98 or 100 mg/ml, preferably about 10 to 80 mg/ml, and most preferably about 45 mg/ml.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof, and
(b) 1 to 150 mM of an acetate buffer solution, and preferably the pH of the pharmaceutical composition is about 2.0 to 6.0.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof, and
(b) 1 to 150 mM of an acetic acid-sodium acetate buffer solution, and preferably the pH of the pharmaceutical composition is about 2.0 to 6.0.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetate buffer solution, and
(c) 2 to 100 mg/ml of mannitol, and preferably the pH of the pharmaceutical composition is about 2.0 to 6.0.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetic acid-sodium acetate buffer solution, and
(c) 2 to 100 mg/ml of mannitol, and preferably the pH of the pharmaceutical composition is about 2.0 to 6.0.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof, and
(b) 1 to 150 mM of an acetate buffer solution, and preferably the osmotic pressure of the pharmaceutical composition is about 280 to 320 mOsmol/kg.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetate buffer solution, and
(c) 2 to 100 mg/ml of a saccharide, and preferably the osmotic pressure of the pharmaceutical composition is about 280 to 320 mOsmol/kg.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetate buffer solution, and
(c) 2 to 100 mg/ml of mannitol, and preferably the osmotic pressure of the pharmaceutical composition is about 280 to 320 mOsmol/kg.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetic acid-sodium acetate buffer solution, and
(c) 2 to 100 mg/ml of mannitol, and preferably the osmotic pressure of the pharmaceutical composition is about 280 to 320 mOsmol/kg.

In alternative embodiments, the pharmaceutical composition of the present disclosure comprises:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetic acid-sodium acetate buffer solution, and preferably the pH of the pharmaceutical composition is about 2.0 to 6.0, and
(c) 2 to 100 mg/ml of mannitol, and preferably the osmotic pressure of the pharmaceutical composition is about 280 to 320 mOsmol/kg.

The pharmaceutical acceptable salt of the active ingredient of the present disclosure is a pharmaceutical acceptable salt selected from the group consisting of hydrochloride, phosphate and citrate, and preferably hydrochloride. Its non-limiting examples include dihydrochloride, trihydrochloride and the like.

In alternative embodiments, the pharmaceutical composition of the present disclosure comprises 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid hydrochloride, and the osmotic pressure of the pharmaceutical composition is about 280 to 320 mOsmol/kg.

In alternative embodiments, the pharmaceutical composition of the present disclosure comprises 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid hydrochloride, and the pH of the pharmaceutical composition is about 2.0 to 6.0.

The present disclosure also provides a method for preparing the above pharmaceutical composition, comprising a step of mixing 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof and the buffer solution. Further, the method also comprises a step of adding the saccharide.

In some embodiments, the pharmaceutical composition is stable at 2 to 8°C for at least 3 months, at least 6 months, at least 9 months, at least 12 months, at least 18 months or at least 24 months. In some embodiments, the pharmaceutical composition is stable at 25±2°C/60±5% RH for at least 7 days, at least 14 days, at least 1 month, at least 2 months, at least 3 months or at least 6 months.

In alternative embodiments, after storing the pharmaceutical composition at 2 to 8°C for 3 months, the growth rate of the total impurity content is not higher than 20%, and can be 20, 18, 16, 14, 12, 10, 9, 8, 7, 6, 5, 4, 3, 2, 1% or lower.

In alternative embodiments, after storing the pharmaceutical composition at 2 to 8°C for 6 months, the growth rate of the total impurity content is not higher than 20%, and can be 20, 18, 16, 14, 12, 10, 9, 8, 7, 6, 5, 4, 3, 2, 1% or lower. In alternative embodiments, after storing the pharmaceutical composition at 2 to 8°C for 9 months, the growth rate of the total impurity content is not higher than 20%, and can be 20, 18, 16, 14, 12, 10, 9, 8, 7, 6, 5, 4, 3, 2, 1% or lower. In alternative embodiments, after storing the pharmaceutical composition at 25±2°C/60±5% RH for 1 month, 2 months, 3 months or 6 months, the growth rate of the total impurity content is not higher than 20%, and can be 20, 18, 16, 14, 12, 10, 9, 8, 7, 6, 5, 4, 3, 2, 1% or lower. The present disclosure also provides a use of the above pharmaceutical composition in the preparation of a medicament for treating or preventing a KOR receptor-related disease or condition in mammals, wherein the KOR receptor-related condition is selected from the group consisting of pain, inflammation, itching, edema, hyponatremia, hypokalemia, intestinal obstruction, cough and glaucoma.

In non-limiting embodiments, the pain of the present disclosure is selected from, but not limited to, neuropathic pain, trunk pain, visceral pain, skin pain, arthritic pain, kidney stone pain, uterine cramp, dysmenorrhea, endometriosis, dyspepsia, post-surgical pain, post-medical treatment pain, eye pain, otitis pain, fulminant cancer pain and GI disorder-related pain.

### Terms

"Buffer solution" refers to a buffer solution that can withstand pH changes through the action of its acid-base conjugated components. Examples of the buffer solution that can control the pH in an appropriate range include acetate, succinate, gluconate, histidine, oxalate, lactate, phosphate, citrate, tartrate, fumarate, glycylglycine and other organic acid buffer solutions.

"Citrate buffer solution" is a buffer solution comprising citrate ions. Examples of the citrate buffer solution include citric acid-sodium citrate, citric acid-potassium citrate, citric acid-calcium citrate, citric acid-magnesium citrate and the like. A preferred citrate buffer solution is citric acid-sodium citrate.

"Succinate buffer solution" is a buffer solution comprising succinate ions. Examples of the succinate buffer solution include succinic acid-sodium succinate, succinic acid-potassium succinate, succinic acid-calcium succinate and the like. A preferred succinate buffer solution is succinic acid-sodium succinate.

"Phosphate buffer solution" is a buffer solution comprising phosphate ions. Examples of the phosphate buffer solution include disodium hydrogen phosphate-sodium dihydrogen phosphate, disodium hydrogen phosphate-potassium dihydrogen phosphate and the like. A preferred phosphate buffer solution is disodium hydrogen phosphate-sodium dihydrogen phosphate.

"Acetate buffer solution" is a buffer solution comprising acetate ions. Examples of the acetate buffer solution include acetic acid-sodium acetate, histidine acetate, acetic acid-potassium acetate, acetic acid-calcium acetate, acetic acid-magnesium acetate and the like. A preferred acetate buffer solution is acetic acid-sodium acetate.

"Pharmaceutical composition" refers to a mixture comprising one or more compound(s) according to the present disclosure or a physiologically/pharmaceutically acceptable salt or produg thereof and other chemical components such as physiologically/pharmaceutically acceptable carriers and excipients. The pharmaceutical composition is intended for maintaining the stability of the antibody active ingredient, promoting the administration to an organism, facilitating the absorption of the active ingredient and thereby exerting the biological effect. As used herein, "pharmaceutical composition" and "formulation" are not mutually exclusive.

The term "about" used in the present disclosure refers to an error range of ±10%.

The typical criterion of acceptable stability according to the present disclosure is as follows: usually no more than about 10%, preferably no more than about 5% of the active ingredient is degraded, determined by HPLC.

The pharmaceutical excipients and reagents involved in the present disclosure are all commercially available. The active ingredient compound A: 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid can be prepared according to the examples of WO2017211272.

Test conditions of the instruments used in the experiments of the present disclosure:
The structures of the compounds were identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a FINNIGAN LCQAd (ESI) mass spectrometer (manufacturer: Thermo, type: Finnigan LCQ advantage MAX).

High performance liquid chromatography (HPLC) was determined on an Agilent 1200DAD high pressure liquid chromatograph (Sunfire C18 150×4.6 mm column) and Waters 2695-2996 high pressure liquid chromatograph (Gimini C18 150×4.6 mm column).

### DETAILED DESCRIPTION OF THE INVENTION

The present disclosure will be further described in detail with reference to the following examples and experimental examples. These examples and experimental examples are for illustrative purposes only, and should not be considered as limiting the scope of the present disclosure.

### Example 1: Preparation of compound A acetate

Compound A (300 mg, 0.424 mmol) was added to dichloromethane (3 mL), and the mixture was stirred to make it clear. A pre-formulated solution of acetic acid (26.7 mg, 0.445 mmol) in dichloromethane (0.5 mL) was added, and the reaction solution was heated to 50°C and stirred for 2 hours under a slight boiling condition. Isopropyl ether (3 mL) was added, and the reaction solution was stirred at room temperature for 2 hours. The reaction solution was filtered, the filter cake was rinsed with isopropyl ether (5 mL×2), and dried under vacuum to obtain the title product (300 mg, yield: 92%).

The ¹H-NMR results of the resulting product are shown below, and the NMR data indicates that in the resulting salt, the molar ratio of the main ingredient to acetic acid is 1:1.
¹H-NMR (400 MHz, CD₃OD) δ 7.40-7.12 (m, 10H), 4.88-4.80 (m, 1H), 4.61 (dd, 1H), 4.39 (dd, 1H), 3.97-3.69 (m, 4H), 3.23-3.06 (m, 3H), 2.98-2.73 (m, 4 H), 2.61 (dd, 1H), 2.53 (dd, 1H), 2.20 (d, 2H), 1.91 (s, 3H), 1.87-1.74 (m, 2H), 1.74-1.51(m, 7H), 1.44 (d, 2H), 1.19 (d, 3H), 1.01-0.87 (m, 6H).

### Example 2:

Compound A (300 mg, 0.42 mmol) and dichloromethane (3 mL) were added to a reaction flask, and stirred until compound A was dissolved completely. A pre-formulated solution of acetic acid (76.3 mg, 1.27 mmol) in dichloromethane (0.5 mL) was added dropwise, and the reaction solution was heated to 50°C and stirred for 2 hours. Isopropyl ether (3 mL) was added, and the reaction solution was slowly cooled to room temperature, and stirred at room temperature for 72 hours. The reaction solution was filtered, and the filter cake was rinsed with isopropyl ether (5 mL×2), and dried under vacuum to obtain the title product (300 mg, yield: 92%).

The ¹H-NMR results of the resulting product are shown below, and the NMR data indicates that in the resulting salt, the molar ratio of the main ingredient to acetic acid is 1:2.
¹H-NMR (400 MHz, CD₃OD)δ 7.39-7.13 (m, 10H), 4.83 (m, 1H), 4.61 (dd, 1H), 4.39 (dd, 1H), 3.94-3.65 (m, 4H), 3.27-3.12 (m, 3H), 2.95-2.79 (m, 4 H), 2.70-2.52 (m, 2H), 2.20 (dd, 2H), 1.93 (s, 6H), 1.84-1.55(m, 9H), 1.51-1.40 (d, 2H), 1.20 (d, 3H), 1.01-0.87 (m, 6H).

### Example 3: Preparation of compound A hydrochloride

Compound A (4.08 g, crude) was added to hydrochloric acid (40 mL, 4M) at 0°C, and stirred at room temperature for 2 hours. The reaction solution was concentrated under reduced pressure, and the resulting residue was purified by preparative chromatography to obtain the title product (710 mg, yield: 17%).

The results of high performance ion chromatography (HPIC) of the resulting product showed a chloride ion content of 12.38%, indicating that in the resulting salt, the molar ratio of the main ingredient to hydrochloric acid was 1:3.

### Example 4: Preparation of the citrate

Compound A (300 mg, 0.423 mmol) was dissolved in isopropanol (5 mL). Citric acid monohydrate (356.2 mg, 1.69 mmol) was added, and the reaction solution was heated to 60°C and stirred for 2 hours. The reaction solution was slowly cooled to room temperature and filtered, and the filter cake was collected and dried under vacuum to obtain the title product (260 mg, yield: 66%).

The ¹H-NMR results of the resulting product are shown below, and the NMR data indicates that in the resulting salt, the molar ratio of the main ingredient to citric acid is 1:3.
¹H-NMR (400MHz, CD₃OD)δ 7.39-7.32 (m, 2H), 7.32-7.19 (m, 7H), 7.15 (t, 1H), 4.89-4.84 (m, 1H), 4.75-4.57 (m, 1H), 4.40-4.30 (m, 1H), 3.98-3.78 (m, 5H), 3.76- 3.58 (m, 2H), 3.25-3.00 (m, 4H), 2.98-2.88 (m, 3H), 2.85 (dd, 5H), 2.77 (s, 3H), 2.73 (s, 3H), 2.32-2.06 (m, 2H), 1.87-1.65 (m, 7H), 1.65-1.47 (m, 2H), 1.43 (brs., 2H), 1.35-1.26 (m, 3H), 1.01-0.81 (m, 6H).

### Example 5: Preparation of the phosphate

Compound A (308 mg, 0.436 mmol) was dissolved in isopropanol (5 mL). Phosphoric acid (200 mg, 1.73 mmol, purity: 85%) was added, and the reaction solution was heated to 60°C and stirred for 2 hours. The reaction solution was slowly cooled to room temperature, and stirred for 16 hours. The reaction solution was filtered, and the filter cake was collected and dried under vacuum to obtain the title product (520 mg, yield: 51.9%).

The results of high performance ion chromatography (HPIC) of the resulting product showed a phosphate ion content of 31.43%, indicating that in the resulting salt, the molar ratio of the main ingredient to phosphoric acid was 1:3.

### Example 6: Stability study

The samples of the acetate, hydrochloride, citrate and phosphate of compound A were spread flat in the air respectively to test the stability under conditions of room temperature, heating (40°C and 60°C), lighting (4500 Lux) and high humidity (RH 75% and RH 90%). The study period was 20 days.

| | | Acetate (1:1) | Acetate (1:2) | Hydrochloride (1:3) | Citrate (1:3) | Phosphate (1:3) |
|---|---|---|---|---|---|---|
| Condition | Time/day | Purity% | Purity% | Purity% | Purity% | Purity% |
| 4500 Lux | 0 | 97.65 | 97.70 | 96.50 | 97.39 | 96.32 |
| | 5 | 96.99 | 95.83 | 95.57 | 97.29 | 96.07 |
| | 10 | 96.68 | 94.91 | 94.27 | 97.28 | 95.62 |
| | 20 | 96.72 | 92.17 | 94.24 | 97.28 | 95.76 |
| 40°C | 5 | 96.23 | 86.31 | 96.54 | 97.10 | 96.12 |
| | 10 | 96.30 | 81.31 | 96.42 | 97.00 | 96.17 |
| | 20 | 95.70 | 66.14 | 96.41 | 97.00 | 96.19 |
| 60°C | 5 | 96.11 | 79.36 | 96.42 | 91.27 | 95.89 |
| | 10 | 95.73 | 71.68 | 96.43 | 89.97 | 95.66 |
| | 20 | 94.97 | 56.02 | 96.41 | 83.88 | 95.82 |

The results showed that the acetate (1:1 or 1:2) degraded to varying degrees under conditions of lighting, 40°C and 60°C, indicating a poor stability. After the completion of the 20-day experiment, the results of the ion chromatography of the sample under the condition of high temperature showed a great decrease in acetate content. In contrast, the phosphate, hydrochloride and citrate had good stability, and are conducive to the further development of pharmaceutical composition (formulation) of compound A that facilitates administration.

### Example 7:

4-Amino-*N*-[*N²*-[*N*-[*N*-[*N*-((*R*)-2-phenylpropyl)glycyl]-D-phenylalanyl]-D-leucyl]-D-lysyl]piperidine-4-carboxylic acid hydrochloride (1:3) was formulated in a buffer solution according to the prescription amount in Table 1. After stirring well and adjusting the pH, the solution was ready for use. The stability under conditions of 25°C and 40°C was studied respectively. The data is shown in Table 2:

**Table 1**

| **Prescription/amount** | **1** | **2** | **3** |
|---|---|---|---|
| Compound A hydrochloride (1:3) | 0.5 mg/ml | | |
| Buffer system | Citric acid-sodium citrate | Acetic acid-sodium acetate | Disodium hydrogen phosphate-sodium dihydrogen phosphate |
| Buffer salt concentration | 40 mM | | |
| pH | 4.5 | 4.5 | 7.0 |

**Table 2**

| **Test item** | **Buffer system** | **0 day** | **40°C** | | **25°C** |
|---|---|---|---|---|---|
| | | | **7 days** | **14 days** | **1 month** |
| Total impurity% | Citric acid-sodium citrate | 0.0 | 0.2 | 0.4 | 0.0 |
| | Acetic acid-sodium acetate | 0.1 | 0.0 | 0.0 | 0.1 |
| | Disodium hydrogen phosphate-sodium dihydrogen phosphate | 0.1 | 0.6 | 0.6 | 0.4 |

Conclusion: In the solution system, the related substance of compound A increased significantly. For example in the disodium hydrogen phosphate-sodium dihydrogen phosphate buffer system, the related substance increased to 0.4% after being stored at 25°C for one month, indicating that the pharmaceutical composition has a poor stability and is not conducive to long-term storage. In contrast, in the acetic acid-sodium acetate buffer system, compound A showed a good stability after being stored at 25°C for one month or after being stored at 40°C for 7 days.

### Example 8: Screening of acetate concentration

A prescription formulation of 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid hydrochloride (1:3) was formulated according to Table 3. The stability under conditions of 40°C and 25°C was studied respectively. The data is shown in Table 4:

**Table 3**

| **Prescription/amount** | **1** | **2** | **3** | **4** |
|---|---|---|---|---|
| Compound A hydrochloride (1:3) | 0.5 mg/ml | | | |
| Acetic acid-sodium acetate concentration | 10 Mm | 20 mM | 40 mM | 80 mM |
| pH | 4.5 | | | |

**Table 4**

| **Test item** | **Acetate concentration mM** | **0 day** | **40°C** | **40°C** | **25°C** |
|---|---|---|---|---|---|
| | | | **7 days** | **14 days** | **1 month** |
| Total impurity% | 10 | 0.2 | 0.0 | 0.0 | 0.0 |
| | 20 | 0.0 | 0.0 | 0.2 | 0.0 |
| | 40 | 0.1 | 0.0 | 0.0 | 0.1 |
| | 80 | 0.0 | 0.2 | 0.3 | 0.3 |
| Content% | 10 | 100.9 | 101.8 | 101.8 | 101.3 |
| | 20 | 100.1 | 100.0 | 99.5 | 101.8 |
| | 40 | 104.8 | 104.7 | 105.2 | 105.2 |
| | 80 | 104.7 | 103.6 | 104.0 | 105.0 |
| pH | 10 | 4.50 | 4.76 | 4.78 | 4.78 |
| | 20 | 4.51 | 4.76 | 4.77 | 4.80 |
| | 40 | 4.50 | 4.55 | 4.55 | 4.56 |
| | 80 | 4.51 | 4.60 | 4.61 | 4.64 |

It can be seen from the test results that compound A can maintain a good stability in different concentrations of acetate buffer solution.

### Example 9:

A prescription formulation of 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid hydrochloride (1:3) was formulated according to Table 5. The stability under conditions of 40°C and 25°C was studied respectively.

**Table 5**

| **Ingredient/amount** | **1** | **2** | **3** |
|---|---|---|---|
| Compound A hydrochloride (1:3) | 0.5 mg/ml | | |
| Acetate concentration | 40 mM | | |
| pH | 4.5 | | |
| Mannitol | 45 mg/ml | N/A | N/A |
| Sodium chloride | N/A | 7 mg/ml | N/A |

**Table 6**

| **Test item** | **Osmotic pressure regulator** | **0 day** | **40°C** | | **25°C** |
|---|---|---|---|---|---|
| | | | **7 days** | **14 days** | **1 month** |
| Total impurity% | None | 0.1 | 0.0 | 0.0 | 0.1 |
| | Mannitol | 0.0 | 0.2 | 0.3 | 0.2 |
| | Sodium chloride | 0.0 | 0.0 | 0.0 | 0.0 |
| Content% | None | 104.8 | 104.7 | 105.2 | 105.2 |
| | Mannitol | 100.2 | 99.6 | 99.1 | 100.6 |
| | Sodium chloride | 101.9 | 101.4 | 101.8 | 102.7 |
| Osmotic pressure mOsmol/kg | None | 49 | N/A | N/A | N/A |
| | Mannitol | 297 | N/A | N/A | N/A |
| | Sodium chloride | 289 | N/A | N/A | N/A |

It can be seen from the test results that the pharmaceutical composition has a good stability with or without the addition of the osmotic pressure regulator, but in order to avoid or reduce the irritation of administration, it needs to add the osmotic pressure regulator to maintain the isotonicity of the pharmaceutical composition, meanwhile considering the issue of the patient's blood sodium during clinical use, mannitol is preferred as the osmotic pressure regulator.

### Example 10:

| | |
|---|---|
| Compound A hydrochloride | 0.5 mg/ml |
| Buffer salt concentration | 40 mM |
| Mannitol | 54 mg/ml |
| pH | 4.7 |

A solution of 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid hydrochloride (1:3) was formulated according to the above prescription amount, and then sterilized and packed. The solution was stored under conditions of 25±2°C, humidity 65±5% (accelerated condition) or 2 to 8°C respectively to study the stability. The specific data are shown in Table 7.

**Table 7**

| Test item | 0 day | 25±2°C/65±5%RH | | | | 2∼8°C | | |
|---|---|---|---|---|---|---|---|---|
| | | 1 month | 2 months | 3 months | 6 months | 3 months | 6 months | 9 months |
| pH | 4.7 | 4.7 | 4.5 | 4.7 | 4.7 | 4.7 | 4.7 | 4.7 |
| Total impurity% | 0.7 | 0.7 | 0.9 | 0.9 | 0.8 | 1.0 | 0.8 | 0.9 |

## Claims

1. A pharmaceutical composition, comprising the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof and a buffer solution, which is preferably an acetate buffer solution, and more preferably an acetic acid-sodium acetate buffer solution.

2. The pharmaceutical composition according to claim 1, wherein the pH of the pharmaceutical composition is about 2.0 to 6.0, and preferably about 3.0 to 5.0.

3. The pharmaceutical composition according to claim 1 or 2, wherein the concentration of the buffer solution is about 1 to 150 mM, preferably about 10 to 80 mM, and most preferably about 20 mM.

4. The pharmaceutical composition according to claims 1 to 3, wherein the concentration of the active ingredient or a pharmaceutically acceptable salt thereof is about 0.1 to 1.0 mg/ml.

5. The pharmaceutical composition according to any one of claims 1 to 4, wherein the osmotic pressure of the pharmaceutical composition is about 280 to 320 mOsmol/kg.

6. The pharmaceutical composition according to any one of claims 1 to 5, further comprising a saccharide, which is preferably mannitol.

7. The pharmaceutical composition according to claim 6, wherein the concentration of the saccharide is about 2 to 100 mg/ml, preferably about 10 to 80 mg/ml, and most preferably about 45 mg/ml.

8. The pharmaceutical composition according to any one of claims 1 to 7, comprising:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof,
(b) 1 to 150 mM of an acetic acid-sodium acetate buffer solution, and
(c) 2 to 100 mg/ml of mannitol, and preferably the pH of the pharmaceutical composition is about 2.0 to 6.0.

9. A pharmaceutical composition, comprising:
(a) 0.1 to 1.0 mg/ml of the active ingredient 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof, and
(b) 1 to 150 mM of an acetic acid-sodium acetate buffer solution.

10. The pharmaceutical composition according to any one of claims 1 to 9, wherein the pharmaceutical acceptable salt of the active ingredient is selected from the group consisting of hydrochloride, phosphate and citrate, and preferably hydrochloride.

11. A method for preparing the pharmaceutical composition according to any one of claims 1 to 10, comprising a step of mixing 4-amino-*N*-[*N²*-[*N*-[*N*-[*N*-((*R*)-2-phenylpropyl)glycyl]-D-phenylalanyl]-D-leucyl]-D-lys yl]piperidine-4-carboxylic acid or a pharmaceutical acceptable salt thereof and the buffer solution.

12. Use of the pharmaceutical composition according to any one of claims 1 to 10 or the pharmaceutical composition prepared by the method according to claim 11 in the preparation of a medicament for treating or preventing a KOR receptor-related disease or condition in mammals.

13. The use according to claim 12, wherein the KOR receptor-related condition is selected from the group consisting of pain, inflammation, itching, edema, hyponatremia, hypokalemia, intestinal obstruction, cough and glaucoma.

14. The use according to claim 13, wherein the pain is selected from the group consisting of neuropathic pain, trunk pain, visceral pain, skin pain, arthritic pain, kidney stone pain, uterine cramp, dysmenorrhea, endometriosis, dyspepsia, post-surgical pain, post-medical treatment pain, eye pain, otitis pain, fulminant cancer pain and GI disorder-related pain.
